# EUROPEAN PATENT APPLICATION

(11) **EP 1 692 999 A1**
(43) Date of publication of application: **23.08.2006**
(21) Application number: 06003573.0
(22) Date of filing: 22.02.2006
(51) Int. Cl.: A61B 5/00

(54) **Sample fluid collecting device**

(30) Priority: 22.02.2005 JP 2005046205
(71) Applicant: Maruishi & Co., Ltd., Suginami-ku Tokyo (JP); Qualitas Inc., Tokyo (JP)
(72) Inventor: Ishida, Katsushi, Suginami-ku Tokyo (JP); Tsubota, Kazuo, Chiba-ken (JP)
(74) Representative: Wolff, Felix

(57) **Abstract**

A sample fluid collecting device includes a capillary tube for collecting a sample fluid; and an air reservoir portion 5 having an air hole 10 capable of being closed, which is located at one end of the capillary tube, the capillary tube being formed by two or more supporting members 2, 3 arranged with one or more gap portions 1 being provided in the lengthwise direction, and at least two transparent films 6, 9 lapped on the supporting members 2, 3 so as to cover the gap portion 1.

## Description

### FIELD OF INVENTION

The present invention relates to a sample fluid collecting device for collecting a sample fluid such as tears, blood, or salvia obtained from a living body.

### BACKGROUND OF THE INVENTION

Very small amounts of sample of fluid such as tears, salvia, or blood one taken from a subject, and such sample are tested for diseases fluid. In particular, in the diagnosis of dry eye, it is important to know the quantity and components of tears.

In order to collect tear fluid samples, the use of capillary tube can be thought of. For example, Japanese Patent Provisional Publication No. 2002-131314 discloses capillary device which collects a gingival crevice fluid etc as a sample fluid to analyze a component in the sample fluid. This capillary device is configured so as to have a substance that reacts with the sample fluid by catalytic action and to place a very small amount of the sample on an enzyme test paper (glucose test paper) used for analysis at a position where the test paper comes into contact with the sample.

A capillary fluid collecting base portion described in the above patent application is used in such a manner that the enzyme test paper is impregnated with a gingival crevice fluid (sample fuid)for subsequent analysis. Generally, the test paper has a stronger suction force for sucking the sample fluid than the capillary . tube, and therefore the sample fluid flows into the rest paper without requiring any means therefore ,so that a mechanism for exhausting the collected is not provided. Therefore, there is a fear that the sample cannot be placed successfully in the sensor portion or on the inspection chip of a measuring instrument that does not use a test paper. Usually, since the suction force dueto capillary action is greater than the force of gravity, the sample fluid in the capillary tube does not come out without using some kind of means to bring the fluid out

Also, even if an attempt is made to take a sample from tears in the eye or a small amount of oozing blood using a pipette, since it is difficult to finely regulate the suction amount, air is undesirably sucked in, and hence the sample fluid may dry up, or it is difficult to exhaust the sample from the pipette without waste. Further, when a pipette is used, volume measurement of a very small amount of tears,which is required at the time of dry eye diagnosis, cannot be made.

Furthermore, a sample fluid collecting device that has a rubber ball member at one end of the capillary tube is known However, when such device is used, if the rubber ball member is touched slightly or its temperature is changed even slightly, the volume of rubber ball member varies. As a result, a very small amount of sample fluid entering into a capillary tube portion is pushed out or sucked inadvertently; so that the volume measurement of sample fluid cannot be made exactly.

### SUMMARY OF THE INVENTION

An object of the prsent invention is to provide a sample fluid collecting device which can collect a sample fluid quickly and enables exact measurement of the volume of sample fluid.

The present invention provides a sample fluid collecting device capable of quickly collecting a sample fluid such as tears, saliva, or blood. More specifically, the present invention provides a sample fluid collecting device including a capillary tube for collecting a sample fluid, and an air reservoir portion having an air hole capable of being closed, which is located at on end of the capillary tube. The capillary tube can be formed by two or more surpporting member arranged with one or more gap portions being provided in the lenthwise direction; and at least two transparent films lapped on the supporting members so as cover the gap portion, and also can be formed by a supporting member having a groove provided in the lengthwise direction; and at least one transparent film lapped on the support member so as to cover the groove.

Also, the air reservoir portion can be formed between the transparent films by increasing the thicknesses of the supporting members and the width of the gap portion at one end of the supporting members, and also can be formed with an elastic resin-made frame body being provided between the supporting member and the transparent film.

Further, the capillary tube can be molded as an integral tube by using a resin. The air reservoir a portion preferably has a flat plate shape so as to be suitable for being held by fingers. It is preferable that the surface area of the tip end portion be increased by widening the width of the supporting member while the thickness of the capillary tube is kept almost the same in the tip end portion on the side opposite to the air reservoir portion of the support member, or a soft material be used in the tip end portion be covered with such a material so that even if a sensitive part of the eye touches the tip end of the sample fluid collecting device, the stimulation is little.

The supporting member or the transparent film is preferably printed or marked with a scale. The supporting member and the transparent film are preferably joined to each other with a hydrophilic adhesive or a pressure-sensitive adhesive.

The above-described tear fluid sample collecting device has a mode in which partitions or slits for dividing the interior of a capillary tube in the width direction are provided in a portion in which the capillary tube takes the sample fluid. The reason for the is to improve the initial suction of sample fluid by decreasing the effective diameter of the capillary tube. Also, a mode in which the widths of openings of the partitions or the slits are different from each other, a mode in which the passages, the partitions, or the slits are formed by a laser beam machining technique, a mode in which differently colored coloring substances are placed in the passages, or a mode in which a hydrophilic adhesive is contained between the supporting member and the transparent film is preferable.

The air reservoir portion of the device in accordance with the present invention is provided with an air hole having a size capable of being easily closed by a finger. Because of the presence of this air hole, the sample fluid ran be sucked quickly into the capillary tube, and further the volume of air reservoir portion is changed by the pressing by a finger or the temperature of finger,so that the sample fluid can be prevent from being sucked or pushed out inadvertently Furthermore, the air reservoir portion has a flat plate shape so as to be easily picked by fingers etc. Therefore, by providing the air hole in this flat surface, the air hole can easily be closed with one hand without much effort, and also the collected sample fluid can be exhausted easily or quantitatively.

At least a part of the inner surface of capillary tube is required to use a hydrophilic material having high wettability with respect to the sample fluid For example, even if a member having rather low hydrophilic properties is used as the supporting member, a hydrophilic material such as a resin film, which is subjected to surface treatment for providing hydrophilic properties, must be used as the transparent film to successfully suck up the sample fluid by utilizing capillary action If the whole of inner surcface of the capillary tube portion is non-hydrophilic or hydrophobic, the sucking-up effect of sample fluid due to capillary action would be too weak However, if a part thereof is hydrophilic, capillary action takes place. If the whole of inner surface of the capillary tube portion is hydrophilic, capillary action preferably becomes stronger so that the sample fluid can be collected more quickly.

Also, the tip end portion of the device that touches the subject when the sample fluid is collected preferably has a round shape (semicircular shape, elliptical shape, or other shape without an angle) to prevent unnecessary stimulation. For this purpose, the width in the tip end portion of supporting member can be increased, the shape of the tip end portion can be made circular, or a ball having a hole with almost the same diameter or width as that capillary tube portion can be attached to the tip end portion.

Further, a coloring substance can be applied or placed in the interior of the capillary tube portion so that the collected sample fluid can be easily visually confirmed. As this coloring substance, a coloring substance for food, fur example, Blue No. 1 or Red No. 102 or a coloring substance for other applications can be used. In place of the use of coloring substance, the inner surface of transparent film can be made rough in a frosted glass form to remove the fogginess of frosted glass by the collected sample fluid, by which the tip end position of sample fluid can be made visible. By doing this, the coloring substance is not mixed with the sample fluid, and a wider-range inspection method such as the colorimetric method can be used.

As the supporting member or the transparent film, a material such as PET, PVA, HEMA (2-hydroxyethyl methacrylate), a polymer modified by a hydrophilic group, semisolid gel, or a titanium oxide contaminating resin, or a resin material subjected to surface treatment by using a hydrophilic functional group or a surface active agent can be used These materials can further be subject, as necessary, to various chemical or physical treatments for providing hydrophilic properties to persons skilled in the art

When thinking of an application for collecting tear fluid from the sensitive eye meniscus the tip end of the sample fluid collecting device is preferably formed of a flexlible material However, even though suitable in terms of flexibility, the material resin does not necessarily have hydrophilic propenies in some cases. Also, in some case, it is difficult to perform treatment for providing hydrophilic properties In such cases, even if the material of a part constituting the capillary tube is not hydrophilic, for example; even if the supporting member is not hydrophilic, if a material forming other parts, for example the transparent film, is hydrophilic, at least two surfaces facing each other four surfaces in the capillary tube are hydrophilic. Therefore, capillary action takes place, and the sample fluid is drawn well into the tube. Examples of a material having flexibility include urethane, silicone rubber, ultra-soft rubber (semisolid gel), water absorbing properties of a resin such as PE, PP, or PET.

The above-described sample fluid collecting device in accordance with the present invention has a dropper function of forcedly exhausting the sample fluid flowing into the capillary tube by closing the air hole with a finger and pressing the air resavoir portion Therefore a very small amount of sample fluid in the capillary tube can be placed easily without waste on an inspection clip made on a substrate such as gass or silicon by microfabrication or in a sample introducing groove in the inspection chip.

Also, a viscous sample fluid that is difficult to suck up can also be collected or exhausted by closing the air hole from the first as if a pipette is used. Further, the inspection IC chip can be incorporated in the capillary tube or at the outlet from the capillary tube to the air reservoir portion. For example, the IC chip is placed in a portion dose to the tip end of the capillary tube, by which a viscous body fluid such as saliva is forcedly sucked up by utilizing capillary action of the pipette function, and the sample fluid is brought into contact with the IC chip. The inspection result data from the IC chip can be displayed by using an external device to read the electric waves.

According to the sample fluid collecting device in accordance with the present invention, a sample fluid can be collected quickly by capillary action, and the volume of the collected sample fluid can be measured exactly and the collected sample fluid can be exhausted easily. Also, according to the device in accordance with the present invention, by the dropper function of this device, the collected sample fluid can be discharged or dripped by an arbitrary amount.

### BRIEF DESCRIPTION OF THE DRAWING

Figure 1 is an exploded view ofa sample fluid collecting device figure 1 is an exploded view of a sample fluid collecting device in accordance with a first embodiment of the present invention.
Figure 2 is an explanatory view of the usage of a sample fluid collecting device in accordance with a first Embodiment of the present invention.
Figure 3 is an exploded view of a sample fluid rollecting device in accordan with a second embodiment of the present invention.
Figure 4 is a sectional view of a sample fluid collecting device in accordance with a third embodiment of the present invention.
Figure 5 is a perspective view of a sample fluid collecting device in accordance with a fourth embodiment of the invention.
Figure 6 is an exploded view showing an example in which an inspection IC chip is arranged in a sample fluid collecting device in accordance with the present invention.
Figure 7 is a schematic view showing a system for analyzing components contained in a sample fluid collected by using a sample fluid collecting device used to carry out the present invention.
Figure 8 is a schematic view showing the construction of an eye and the place where a tear fluid, which is one of sample fluids, is present.

### DETAILED DESCRIPTION OF THE PREFERRED EMBODIMENT OF THE INVENTION

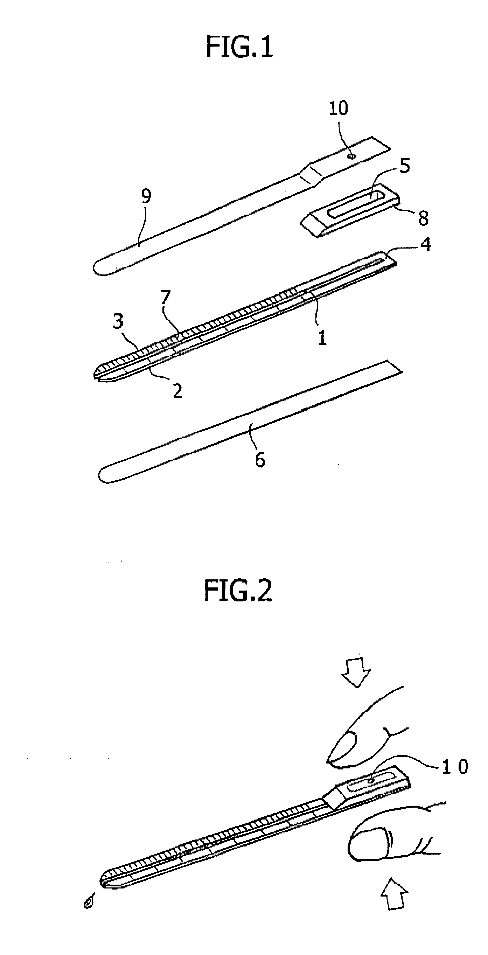

Figure 1 is an exploded view of a sample fluid collecting device in accordance with a first embodiment, of the present invention. As shown in Figure 1, the sample fluid collecting device includes supporting members 2 and 3 provided so that at least one gap 1 is formed in the lengthwise direction of the supporting member, a supporting member connecting portion 4 for connecting the supporting members 2 and 3 to each other, and transparent films 6 and 9 which are lapped on the supporting members 2 and 3 so as to cover the gap 1 to form a capillary tube for taking a sample fluid. For example, an embodiment in which three supporting members are provided and two gaps are formed can be implemented. At one end of the sample fluid collecting device, a frame body 8 which is formed by a discontinuous foam for froming an air reservoir portion 5 and is formed with a cavity therein is arranged between the transparent film 9 supporting bodies 2, 3 and 4. At a position corresponding to the air reservoir portion 5 of the upper transparent film 9, a small air hole 10 is formed. This air hole 10 can be closed easily with a finger, and has a diameter of, for example, about 0.2 to 3 mm, preferably about 0.5 to 1.5 mm. Also, the width of the cavity in the frame body 5 is preferably greater than the width of a passage forming a capillary tube. However, the width of the cavity is not subject to any special restriction. Since the frame body 8 is somewhat elastic, if the air reservoir portion 5 formed between the transparent film 9 and the supporting bodies 2, 3 and 4 is pushed with two fingers so as to close the air hole 10 as shown in Figure 2, the sample fluid in the capillary tube can be pushed out by the air in the air reservoir position 5. Also, the supporting members 2 and 3 and the transparent films 6 and 9 can be joined to each other with a pressure-sensitive adhesive or an adhesive with the frame body 8 being held therebetween. This joining is preferably airtight. Joining utilizing heat can also be performed. A hydrophilic adhesive, which can regulate the surface energy in a wide range, regulates the wettability between the sample fluid and the hydrophilic adhesive, and hence can control the intensity of capillary action, namely, the uptake rate of sample fluid. The air hole 10 can be formed after or before the joining of the transparent film 9.

The thickness of the supporting member 2, 3 can be set at about 0.1 to 1.5 mm, preferably, about 0.2 to 0.5 mm, and the width of the gap 1 can be set at about 0.3 to 2 mm, preferably about 0.5 to 1.0 mm. If the collection amount of sample fluid is set at about 0.1 µl per 1 mm of scale, the size of capillary tube formed in the sample fluid collecting device can be set at about 0.2 mm wide and 0.5 mm thick. However, for example, by excimer laser processing, a capillary tube with smaller inside dimensions can be formed, and bence the collection amount of sample fluid can be made smaller (1 nl to 0.1 µl per 1 mm of scale). Also, a scale 7 is marked on the supporting member of sample fluid collecting device along the lengthwise direction so that the amount of collected sample fluid can be seen at a glance.

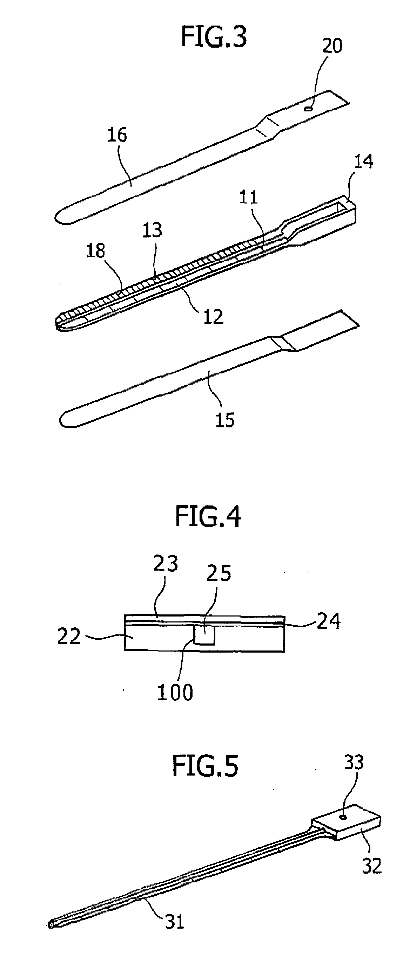

Figure 3 is an exploded view of a sample fluid collecting device in accordance with a second embodiment, which is used to carry out the present invention. This sample fluid collecting device uses supporting bodies 12.13 and 14 formed integrally of a discontinuous foam in place of the supporting bodies 2, 3 and 4 and the frame body 8 in the device shown Figure 1. One end of this supporting body has an increased thickness, and the shape corresponding to the frame body 5 shown in Figure 1 is formed integrally with the supporting bodies 12 and 13. Transparent films 15 and 16 and air hole 20 are the same as the corresponding elements in the device shown in Figure 1. The supporting members 12 and 13 each are provided with scale 18.

Figure 4 is a sectional view of a capillary tube portion of a sample fluid collecting device in accordance with a third embodiment, which is used to carry out the present invention. In this embodiment, a supporting body 22 has a groove in the center thereof, and a transparent film 23 is bonded to above the supporting body 22 with an adhesive 24, by which a capillary tube 25 is formed.

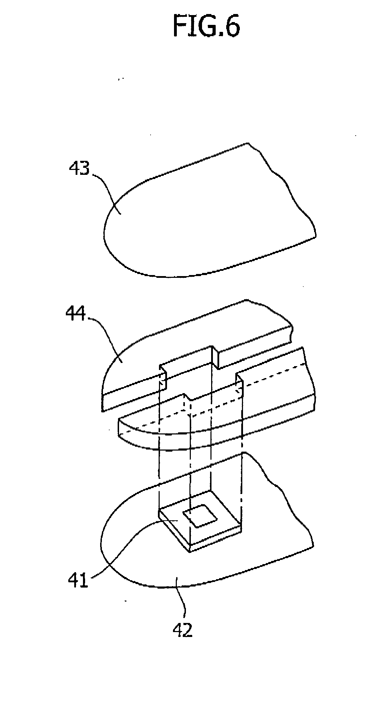

Figure 5 is a sectional view of a sample fluid collecting device in accordance with a fourth embodiment of the present invention, which is formed integrally by utilizing a blow injection molding technique. This sample fluid collecting device can be formed by using a hydroplulic resin. A capillary tube portion 31 is marked or printed with a scale. An air reservoir portion 32 has a flat plate shape so that the air reservoir portion 32 can easily be held between fingers and an air hole 33 communicating with a hollow portion in the air reservoir portion 32 is provided.

Furthermore, an inspection IC chip can be incorporated in the capillary tube or at an outlet of capillary tube to the air reservoir portion or in the vicinity of the outlet. For example, as shown in Figure 6, an inspection IC chip 41 is placed in notches formed in the supporting bodies 44 at positions near the tip end of the capillary tube, and a tear fluid etc can be sucked up forcedly by utilizing capillary action or a viscous body fluid such as saliva can be sucked up forcedly by utilizing the pipette function. This inspection IC chip comes into contact with the sample fluid and forms a state of being capable of forming inspection result data. The IC chip 41 is fixed on a lower transparent film 42, and an upper transparent film 43 can be put on the supporting bodies 44. The inspection result data from the IC chip 41 can be read in a non-contact manner using electric waves by an external device (not shown).

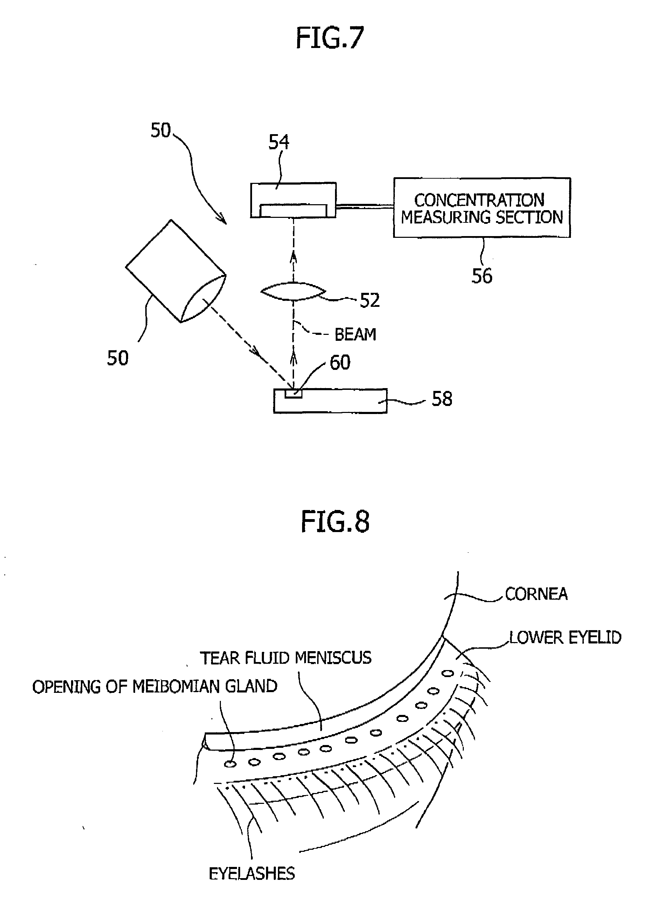

Figure 7 shows a system 50 for optically analyzing components contained in a sample fluid collected by using the sample fluid collecting device used for carrying out the present invention. This system 50 includes a semiconductor laser 50 for emitting beam-form laser beams toward a test paper 58, which is set at a predetermined position, at an incident angle of 45 degrees, a lens 52 for connsing laser beams reflected from the test paper 58, a photodiode 54 for receiving laser beams condensed by the lens 52 and sending an electrical signal corresponding to the amount of received light, and a concentration measuring section 56 for measuring the concentration of a component such as glucose contained in the collected sample fluid based on the electrical signal sent from the photodiode 54 and displaying the concentration.

The system 50 can be used to measure the concetration of a specific component contained in the sample fluid as described below. First, the body fluid collected by the collecting device of each embodiment of the present invention is dripped into the test paper 58. Then, the test paper 58 is set at a predetermined position, and laser beams are emitted toward a body fluid drip portion 60 in the test paper 58, by which the concentration of a component such as glucose contained in the sample fluid is measured optically. The light emitted toward the test paper 60 may be emitted from a light emitting diode (LED).

Also, since the color concentration characteristics of the test paper 58, name1y, the color concentration with respect to the concentration of glucose contained in the sample fluid has a good linearity, the concentration measuring section 56 has a correspondence table showing the corresponding relationship between the electrical signal sent from the photodiode 54 and the concentration of glucose correponding to this electrical signal, which is stored in a storage section of a computer, such as a hard disc or memory. Therefor, if an electrical signal sent: from the photodiode 54 is received, the concetration measuring section 56 looks up the correspondence table, judges the concentration of glucose corresponding to this electrical signal, and displays the concentration value.

Also, a system other than the system 50 shown in Figure 7 can be used to measure a specific conponent in the sample fluid. For example a small-size blood glucose meter (trade name: Glucocard) put on sale by ARKRAY, Inc. or Sanwa Chemical Co, Ltd. can be used to easily measure the concentration of glucose, which is a specific component in the sample fluid. With this meter, electrons delivered when the glucose sample fluid is oxidized by the catalytic action of glucose oxidase are detected by using electrodes (sensor) attached to the meter to determine the concentration of glucose in the sample fluid. For reasons of hygiene, this meter uses disposable type electrodes, and is configured so that calibration is achieved automatically when the electrodes are replaced. The calibration is achieved to exclude an influence due to a difference between individual manufactred electrodes. Also, the amount of sample fluid that can be measured by this meter is about 3 µl.

Next, a method for measuring the concentration of glucose in a sample fluid using the above-described small-size blood glucose meter is explained briefly. First, a tear fluid sample is collected by using the above-described sample fluid collecting device used to carry out the present invention. Next, the tear fluid sample collected by the sample fluid collecting device 1 to 4 is brought into contact with the electrodes attached to the above-described small-size blood glucose meter. Then, a current flows between the electrodes via the sample fluid, and the measurremement of glucose concentration is stanted automatically. The measurement time is about 30 seconds. When the measurement is finished, the glucose concentration is dispalyed in the display section of the small-size blood glucose meter. Also, since this data is stored in the memory in the meter, this data can be transferred to a computer as necessary.

Figure 8 shows the construction of an eye and the place where a tear fluid (tear fluid meniscus), which is one of sample fluids, is present. This figure shows a state in which a tear fluid meniscus (also called a fear triangle) is formed along between a cornea and a lower eyelid. In the tear fluid of this tear fluid meniscus, a negative pressure, which is proportional to the surface tension of the tear fluid and inversely proportional to the radius of curvature thereof due to the principle of capillary action, is produced. Therefore, at the front edge of the tear fluid, a concave surface called a skin mucosa joint portion formed.

A mucous layer containing much polysaccharide such much lies on the comea , and lachrymal water layer and a lipid layer are formed on the mucous layer. The lachrymal water layer connects with the tear fluid meniscus under the lachrymal water layer. Although the amount of tear fluid and lipid in this tear fluid meniscus relate greatly to the state of disease of dry eye, the amounts are difficult to measure because the amounts are very small . The sample fluid collecting device in accordance with the present invention can collect a tear fluid, which is a sample fluid, in a short period of time of about five minutes by placing the tip end thereof at a position of such a degree that the device comes into contact with the tear fluid meniscus at the upper end of the lower eyelid Since the sample fluid can be collected in such a short period time, the sample fluid can be collected sufficiently merely by holding the device by hand or by utilizing a simple holding element without specially fixing the collecting device on a cheek. The amount of the collected tear fluid can be read from the scale of the sample fluid collecting device in accordance with the present invention , and the amount of tear fluid in the tear fluid meniscus can be measured (meniscometry). Such meniscometry using the sample fluid collecting device in accordance with the present invention can be called capillary meniscometry.

The respect in which the tear fluid meniscus amount can be taken in a period of time as short as about five minutes is based on an experiment result that the tear fluid meniscus of a subject was fluorescein dyed and the device in accordance with the present invention was applied to the tear fluid meniscus for five minutes and subsquently light was irradiated, and resultantly light emission fluorescein, which was a dyeng fluid ,was not found

A clearance test for tear fluid can be carried out by using the system shown in Figure 7 or a commercially available small-size blood glucose meter and the sample fluid collecting device in accordance with thepræattinvention. In the current clearance test, a 5% fluorescein solution of about 1 µl per one eye is put in a rotjmct:ival sac, and afta-five minutes, the mlltiple of dî1ution is oomined by using a Sc.hirIm' fa paper. If the tear fluid clearance is poor, foreign matter that has entered the eye will indefinitely remain on the surface of the eye, which will thereby exert an adverse influence on the conjuctiva. According to the present invention, in place of the fluorescein solution, about 1 µl of a sugar solution in which saccharide such as glucose of about 10 grams per 1 liter is put in the eye, and after a preset time, for example, five minutes or ten minutes has elapsed, the tear fluid is sampled to measure the concentration of saccharide in the tear fluid by the system shown in Figure 7 or the commercially available small-size blood glucose meter called Glucocard. According to this method, by using the sample fluid collecting device in accordance with the present invention in combination with the commercially available system, the measurement can be made easily, and also a sense of strangeness experienced by the subject of inspection can be alleviated because no dye is used.

As described above, an explanation has been given of the sample fluid collecting device in accordance with the present invention and the application examples thereof. The present invention is not limited to the above described embodiments, and the present invention can be applied to any sample fluid in addition to tears and saliva in the same way.

## Claims

1. A sample fluid collecting device comprising:
a capillary tube for collecting a sample fluid, and
an air reservoir portion having an air hole capable of being closed, which is located at one end of the capillary tube.

2. The sample fluid collecting device according to claim 1, wherein the capillary tube is formed by two or more supporting members arranged with one or more gap portions being provided in the lengthwise direction; and at least two transparent films lapped on the supporting members so as to cover the gap portion.

3. The sample fluid collecting device according to claim 1, wherein the supporting members are connected to each other at one end.

4. The sample fluid collecting device according to claim 2, wherein the air reservoir portion is formed between the transparent films by increasing the thicknesses of supporting members and the width of the gap portion at one end of the supporting members.

5. The sample fluid collecting device according to claim 1, wherein the capillary tube is formed by a supporting member having groove provided in the lengthwise direction, and at least one transparent film lapped on the support member so as to cover the groove.

6. The sample fluid collecting device according to claim 5, wherein the air reservoir portion is formed between the transparent film and the supporting member by increasing the thickness of supporting member and the depth and width of the groove at one end of the member.

7. The sample fluid collecting to claim 1, wherein the capillary tube is blow molded as an integral tube by using a resin.

8. The sample fluid collecting device according to claim 1, wherein the air reservoir portion has a flat plate shape.

9. The sample fluid collecting device according to claim 2, wherein the width or thickness, or both width and thickness, of the supporting member are large while the thickness of the capillary tube is the same in the tip end portion on the side opposite to the air reservoir portion of the support member.

10. The sample fluid collecting device according to claim 2, wherein the supporting member or the transparent film is printed or marked with a scale.

11. The sample fluid collecting device according to claim 2, wherein a hydrophilic adhesive or a pressure-sensitive adhesive is contained between the supporting member and the transparent film.

12. The sample fluid collecting device according to claim 2, wherein the inner surface of the transparent film is frosted.

13. The sample fluid collecting device according to claim 1, wherein **characterized in that** a water-based coloring substance is placed in the capillary tube.

14. The sample fluid collecting device according to claim 1, wherein an IC chip is placed in the capillary tube.
